# EUROPÄISCHE PATENTSCHRIFT

(11) **EP 3 020 435 B1**
(45) Veröffentlichungstag und Bekanntmachung des Hinweises auf die Patenterteilung: **29.11.2017**
(21) Anmeldenummer: 15176136.8
(22) Anmeldetag: 09.07.2015
(51) Int. Cl.: A61M 15/00, B65D 75/40, B65B 9/02, G01N 35/10

(54) **VERFAHREN UND VORRICHTUNG ZUR KONTROLLIERTEN ABGABE VON PARTIKELN**
METHOD AND DEVICE FOR CONTROLLED EMISSION OF PARTICLES
PROCEDE ET DISPOSITIF DE DEPOT CONTROLE DE PARTICULES

(30) Priorität: 14.11.2014 DE 102014116694
(43) Veröffentlichungstag der Anmeldung: 18.05.2016
(73) Patentinhaber: Bluestone Technology GmbH, 55286 Wörrstadt (DE)
(72) Erfinder: GÖRGEN, Frank, 55268 Nieder-Olm (DE); SEHLINGER, Torsten, 55278 Undenheim (DE)
(74) Vertreter: Manitz Finsterwald Patentanwälte PartmbB

(56) Entgegenhaltungen:
- WO-A1-2010/063714
- US-A- 4 712 460
- US-A- 6 012 454
- US-A1- 2001 010 224
- US-A1- 2005 258 182
- US-B2- 7 837 942

## Beschreibung

Die vorliegende Erfindung betrifft ein Verfahren und eine Vorrichtung zur kontrollierten Abgabe von allergenen Partikeln, um Patienten mit Allergien einer dosierten und kontrollierten Menge an allergenen Partikeln auszusetzen. Indem der Patient unterschiedlichen Allergenen in unterschiedlichen Konzentrationen ausgesetzt wird, kann herausgefunden werden, welche Symptome in welcher Stärke auftreten.

Aus dem Stand der Technik sind verschiedene Verfahren und Vorrichtungen zur Abgabe von allergenen Partikeln bekannt, bei denen die Abgabe der Partikel über einen turbulenten Luftstrom erfolgt, was zu einer diskontinuierlichen und räumlich ungleichmäßigen Verteilung der Partikel führt.

Aus der US 7 837 942 B2 ist ein Verfahren zur Abgabe von allergenen Partikeln bekannt, bei dem allergene Partikel getrennt von einem Luftstrom in eine Messkammer ausgetragen und in der Messkammer turbulent verwirbelt werden. Zum Erfassen der Menge an Partikeln innerhalb der Messkammer werden diese auf verschiedene Art und Weise erfasst.

Aus der US 2001/010224 A1 ist eine Verpackung mit bevorrateten allergenen Partikeln bekannt, bei der die Partikel in einem Behälter mit einer flexiblen Wand untergebracht sind, wobei mehrere Behälter an einem Band angeordnet sind. Die Oberseite der Behälter ist durch einen Filter verschlossen und auf einer Seite des Bandes ist eine Abdeckung angebracht.

Aus der US 2005/258182 A1 ist eine Vorrichtung zur kontrollierten Abgabe von Medikamenten bekannt, bei der einzelne Dosen eines Medikaments auf einem streifenförmigen Träger bevorratet sind.

Die US 6 012 454 A offenbart eine Inhalationseinrichtung für trockenes Pulver, wobei das zu inhalierende Pulver in einzelnen Dosen auf einem bandförmigen Träger bevorratet sein kann.

Es ist die Aufgabe der vorliegenden Erfindung, ein Verfahren und eine Vorrichtung zur kontinuierlichen Abgabe von allergenen Partikeln zu schaffen, mit denen auch bei niedrigen Konzentrationen eine konstante und gleichmäßige Abgabe der allergenen Partikel erfolgen kann.

Die Lösung dieser Aufgabe erfolgt durch die Merkmale der unabhängigen Ansprüche.

Bei dem erfindungsgemäßen Verfahren zur kontrollierten Abgabe von allergenen Partikeln werden zunächst bevorratete Partikel, z.B. natürliche und/oder künstlich hergestellte Partikel, einer Messstation kontinuierlich zugeführt. In der Messstation wird die Menge an zugeführten Partikeln kontinuierlich erfasst, so dass eine sehr genaue Aussage darüber getroffen werden kann, wie viele Partikel in welcher Konzentration pro Zeiteinheit zugeführt werden. Anschließend werden die erfassten Partikel von der Messstation zu einer Austrageinrichtung überführt, aus der die Partikel anschließend abgegeben werden.

Mit dem erfindungsgemäßen Verfahren lässt sich auf sehr präzise Weise kontrollieren und dokumentieren, welche genaue Menge an allergenen Partikeln durch die Austrageinrichtung beispielsweise in einen Raum abgegeben worden ist. Hierdurch lässt sich mit wesentlich höherer Genauigkeit ermitteln, welche Symptome in welcher Stärke auftreten.

Vorteilhafte Ausführungsformen der Erfindung sind in der Beschreibung, der Zeichnung sowie die Unteransprüchen beschrieben.

Nach einer ersten vorteilhaften Ausführungsform können die Partikel der Messstation in einer streifenförmigen Anordnung zugeführt werden. Dies erleichtert nicht nur die spätere kontrollierte Abgabe der Partikel sondern es wird gleichzeitig die Voraussetzung für eine vereinfachte Erfassung der Partikel geschaffen, da diese sich bei Eintritt in die Messstation bereits in einem räumlich begrenzten Bereich, nämlich der streifenförmigen Anordnung, befinden.

Weiterhin kann es vorteilhaft sein, wenn die Partikel der Messstation im Wesentlichen einlagig zugeführt werden, da hierdurch eine exakte Erfassung der zugeführten Partikel erleichtert ist.

Nach einer weiteren Ausbildung der Erfindung können die Partikel der Messstation in einer Verpackung zugeführt werden. Dies besitzt einerseits den Vorteil, dass die Partikel auch noch bei Eintritt in die Messstation gegen Umwelteinflüsse und Beschädigung geschützt sind. Zudem lassen sich die Partikel in der Messstation zuverlässiger erfassen, wenn diese in einer Verpackung angeordnet und somit lagefixiert sind. Hierbei kann es auch vorteilhaft sein, wenn die Partikel in einer Verpackung durch die Messstation hindurchgeführt werden, da hierdurch ausgeschlossen ist, dass bereits erfasste Partikel vor Abgabe durch die Austrageinrichtung verlorengehen.

Nach einer weiteren vorteilhaften Ausführungsform können die Partikel in einer verschlossenen Verpackung in die Austrageinrichtung eingebracht werden, wobei die Verpackung in der Austrageinrichtung bzw. erst in der Austrageinrichtung geöffnet wird. Auf diese Weise ist sichergestellt, dass sämtliche Partikel erst nach Eintritt in die Austrageinrichtung freigesetzt werden, damit die Menge an abgegebenen Partikeln nicht von der Menge an erfassten Partikeln abweicht.

Nach einer weiteren vorteilhaften Ausführungsform können die Partikel in einer streifenförmigen Verpackung zugeführt werden, die insbesondere zwei Filmstreifen umfassen kann, die vorzugsweise transparent oder transluzent sind. Durch eine solche streifenförmige Verpackung lassen sich die Partikel auf besonders einfache Weise kontinuierlich transportieren, wobei während des Transports eine Erfassung der Partikel in der Messstation erfolgen kann. Durch Verwenden einer Verpackung mit zwei licht- oder strahlungsdurchlässigen Filmstreifen kann während des Transports kontinuierlich eine Erfassung der Partikel erfolgen, ohne dass die Verpackung geöffnet werden muss.

Nach einer weiteren vorteilhaften Ausführungsform können die Partikel in der Austrageinrichtung von der Innenseite einer geöffneten Verpackung abgesaugt werden. Auf diese Weise ist sichergestellt, dass die Partikel berührungsfrei jedoch weitegehend aus der Verpackung entfernt werden. Weiterhin kann es vorteilhaft sein, wenn die Partikel mittels Druckluft in der Austrageinrichtung vereinzelt und aus der Austrageinrichtung abgegeben werden. Auf diese Weise wird wiederum berührungsfrei dafür gesorgt, dass sämtliche Partikel vereinzelt abgegeben werden, um eine genau bekannte Anzahl an Partikeln freizusetzen.

Um eine besonders genaue Dosierung der freigesetzten Partikel zu erreichen, kann es vorteilhaft sein, wenn die Geschwindigkeit, mit der die Partikel zu der Austrageinrichtung überführt werden, in Abhängigkeit von der erfassten Menge an Partikeln gesteuert, insbesondere geregelt wird. Wenn somit die Menge an pro Zeiteinheit von der Messeinheit erfassten Partikeln variiert, kann durch Regeln der Zuführgeschwindigkeit die Menge an abgegebenen Partikeln so gesteuert werden, dass diese konstant ist und einer vorgegebenen Dosierung entspricht.

Nach einer weiteren vorteilhaften Ausführungsform können die Partikel in der Messstation einzeln gezählt werden. Hierdurch ist eine sehr genaue Bestimmung der zugeführten Menge an Partikel möglich.

Weiterhin kann es vorteilhaft sein, in der Messstation von den Partikeln Bilddaten zu erfassen. Hierdurch kann eine zuverlässige Dokumentation erstellt werden, welche Partikel, beispielsweise welche Art von Pollen, dem Patienten zugeführt worden sind, um eine Qualitätssicherung zu gewährleisten.

Eine erfindungsgemäße Vorrichtung zur kontrollierten Abgabe von allergenen Partikeln umfasst eine Zuführvorrichtung zum kontinuierlichen Zuführen von bevorrateten Partikeln zu einer Messstation, in der die Menge an zugeführten Partikeln kontinuierlich erfasst wird. Zur Abgabe der erfassten Partikel ist eine Austrageinrichtung vorgesehen, mit der die Partikel insbesondere in einen Raum abgegeben werden.

Bei der erfindungsgemäßen Vorrichtung kann es vorteilhaft sein, wenn die Messstation ein Mikroskop aufweist, um die zugeführten Partikel kontinuierlich und insbesondere unterbrechungsfrei zu erfassen. Hierbei kann es sich beispielsweise um ein Durchlichtmikroskop handeln, durch das die zugeführten Partikel hindurchgeführt werden. Sofern die Messstation eine Zähleinrichtung aufweist, mit der die in die Messstation zugeführten Partikel gezählt werden, ist eine besonders exakte Erfassung der zugeführten Partikel möglich. Eine solche Zähleinrichtung kann beispielsweise mit Hilfe einer elektronischen Kamera erfolgen, die über entsprechende Software eine Zählung der pro Zeiteinheit an der Kamera vorbeigeführten Partikel durchführt.

Nach einer weiteren vorteilhaften Ausführungsform kann die Austrageinrichtung einen Öffnungsmechanismus zum Öffnen einer Verpackung der Partikel aufweisen. Hierdurch wird eine Verpackung der Partikel erst unmittelbar vor dem Freisetzen der Partikel geöffnet, wodurch diese bis zur Freisetzung gegen Umwelteinflüsse und ungewünschte Verteilung geschützt sind.

Die Austrageinrichtung kann zur berührungsfreien Entnahme der Partikel aus der Verpackung eine Absaugeinrichtung aufweisen, welche die Partikel von der Innenseite einer geöffneten Verpackung absaugt. Hierdurch ist sichergestellt, dass kein Partikel ungewünscht an der Innenseite der Verpackung verbleibt, so dass exakt die Menge an erfassten Partikeln durch die Austrageinrichtung abgegeben wird. Hierbei kann es vorteilhaft sein, wenn die Partikel in der Austrageinrichtung mittels einer Düsenanordnung sowohl angesaugt wie auch in vereinzelter Form abgegeben werden.

Zur Bevorratung und zum Transport der allergenen Partikel ist erfindungsgemäß eine Verpackung vorgesehen, die zwei miteinander verbundene Filmstreifen umfasst, zwischen denen sich in einer einlagigen und streifenförmigen Anordnung die allergenen Partikel befinden. Die Filmstreifen sind transparent oder transluzent, so dass die sich zwischen den Filmstreifen befindlichen Partikel mit Hilfe einer optischen Anordnung erfasst und insbesondere gezählt werden können.

Eine vereinfachte Verpackung kann dadurch vorgesehen werden, dass die Partikel in der Verpackung in der streifenförmigen Anordnung zufällig verteilt sind. Hierdurch ist es nicht erforderlich, dass die einzelnen Partikel in der Verpackung geordnet positioniert werden. Dennoch ist ein zuverlässige Erfassung bzw. Zählung der Partikel möglich, indem diese beispielsweise durch ein Mikroskop hindurchgeführt werden. Eine erhöhte Präzision der Zählung erfolgt dadurch, dass die Partikel in der streifenförmigen Anordnung in der Verpackung einlagig angeordnet sind.

Bei einem erfindungsgemäßen Verfahren zur Herstellung einer Verpackung mit bevorrateten allergenen Partikeln werden allergene Partikel zwischen zwei transparenten oder transluzenten Film- oder Folienstreifen streifenförmig oder linienförmig und einlagig angeordnet, woraufhin die beiden Film- oder Folienstreifen zumindest an ihren Rändern miteinander verbunden werden, so dass die Partikel zwischen den Film- oder Folienstreifen eingeschlossen sind.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: eine Seitenansicht einer Vorrichtung zum Herstellen einer Verpackung mit bevorrateten Partikeln;
- Fig. 2: eine Seitenansicht einer Vorrichtung zur kontrollierten Abgabe von Partikeln;
- Fig. 3: eine Schnittansicht durch eine Düsenanordnung entlang der Linie III-III von Fig. 4;
- Fig. 4: eine Seitenansicht der Düsenanordnung von Fig. 3; und
- Fig. 5: eine Austrageinrichtung zur Abgabe von Partikeln.

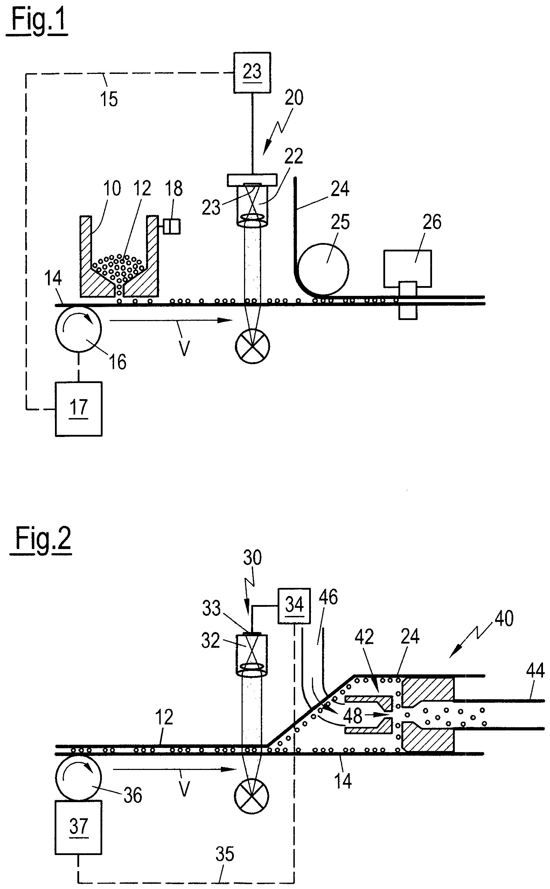

Fig. 1 zeigt in stark vereinfachter schematischer Darstellung eine Vorrichtung zum Herstellen einer Verpackung mit bevorrateten Partikeln, insbesondere allergenen Partikeln, mit der die Partikel in einer streifenförmigen Anordnung zwischen zwei Filmstreifen verpackt werden. Hierzu werden die in einem Reservoir 10 befindlichen allergenen Partikel 12, beispielsweise Pollen, in einer streifenförmigen Anordnung auf einen Filmstreifen 14 abgegeben, der als Träger dient und der über eine Antriebseinrichtung 16 in Richtung des Pfeils V kontinuierlich bewegt wird. Zur Abgabe der Partikel in einer einlagigen oder im Wesentlichen einlagigen Anordnung ist in dem Reservoir 10 eine Austrittsöffnung für die Partikel 12 vorgesehen, durch welche diese vertikal nach unten auf den Filmstreifen 14 abgegeben werden können. Um die Abgabe zu erleichtern, kann das Reservoir 10 mit einer Vibrationseinrichtung und/oder Rühreinrichtung 18 versehen sein, um die Reibung zwischen den einzelnen Partikeln 12 zu reduzieren.

Nach Abgabe der Partikel 12 auf den Filmstreifen 14 in einer kontinuierlichen streifenförmigen oder linienförmigen Anordnung werden diese durch eine Zähleinrichtung 20 geführt, die beim dargestellten Ausführungsbeispiel ein Durchlichtmikroskop 22 umfasst, welches mit einer elektronischen Kamera 23 versehen ist, die mit einer nicht näher dargestellten Recheneinrichtung in Verbindung steht. Auf diese Weise können die auf dem Filmstreifen 14 befindlichen Partikel nicht nur gezählt sondern auch bildtechnisch erfasst werden, um eine gute Qualitätssicherung zu gewährleisten. Gleichzeitig kann durch die Zähleinrichtung 20 über eine Steuerleitung 15 eine Steuerung der Geschwindigkeit eines Antriebs 17 der Antriebsvorrichtung 16 durch einen Regelkreis erfolgen, so dass eine konstante Anzahl an Partikeln pro Längeneinheit des Filmstreifens bereitgestellt wird.

Nach Durchlaufen der Zähleinrichtung 20 wird von der Oberseite des Filmstreifens 14 ein zweiter Filmstreifen 24 zugeführt und über eine Umlenkrolle 25 in die Horizontale umgelenkt, so dass durch Versiegeln der beiden Filmstreifen 14 und 24 in einer Siegelstation 26 eine streifenförmige Verpackung mit bevorrateten Partikeln geschaffen ist, in der sich die Partikel zwischen den beiden Filmstreifen in einer streifenförmigen Anordnung befinden. Die beiden in der Siegelstation 26 an ihren äußeren Rändern miteinander verbundenen Filmstreifen, die beispielsweise durch Hitzeeinwirkung, durch Ultraschalleinwirkung oder durch Klebemittel zumindest an den Rändern miteinander verbunden sind, sind bei der dargestellten Ausführungsform aus einem transparenten Material gebildet und können nachdem Versiegeln für die Aufbewahrung und den Transport zu einer Rolle aufgewickelt werden.

Fig. 2 zeigt in ebenfalls stark schematisch vereinfachter Darstellung eine Vorrichtung zur kontrollierten Abgabe von allergenen Partikeln, umfassend eine Zuführvorrichtung 36 zum kontinuierlichen Zuführen von bevorrateten Partikeln zu einer Messstation 30, wobei in der Messstation 30 die Menge an zugeführten Partikeln 12 kontinuierlich erfasst wird. Nach dem Erfassen werden die Partikel einer Austrageinrichtung 40 zugeführt, in der die erfassten Partikel beispielsweise ein einen Raum abgegeben werden.

Mit Hilfe der Zuführvorrichtung 36, die eine von einem Antrieb 37 angetriebene Antriebsrolle aufweisen kann, wird die wie vorstehend beschrieben hergestellte Verpackung, welche die beiden miteinander verbundenen Filmstreifen 14 und 24 umfasst, in horizontaler Richtung entlang des Pfeils V durch die Messstation 30 hindurchgeführt, die wiederum ein Lichtmikroskop 32 umfasst, das eine elektronische Kamera 33 aufweist, wobei das Lichtmikroskop 32 mit einer Zähleinrichtung 34 verbunden ist, in der die von der Kamera 33 erfassten Partikel fotografiert und einzeln gezählt werden. In der Zähleinrichtung 34 erfolgt also nicht nur ein Zählen der vorzugsweise einlagig durch die Messstation 30 hindurchgeführten Partikel sondern diese werden auch bei dem Durchlaufen der Messstation fotografisch erfasst, so dass Bilddaten der Partikel abgespeichert werden können.

Wie Fig. 2 ferner verdeutlicht, ist die Zähleinrichtung 34 über eine Steuerleitung 35 mit dem Antrieb 37 der Zuführvorrichtung 36 verbunden, so dass die Menge an Partikeln, welche die Messstation 30 verlassen, durch eine rückgekoppelte Regelung konstant gehalten werden kann, indem die Geschwindigkeit der Zuführvorrichtung 36 in Abhängigkeit von der Anzahl an Partikeln, die von der Zähleinrichtung 34 erfasst worden, sind geregelt wird.

Nach Durchlaufen der Messstation 30 werden die Partikel 12 zunächst noch in ihrer Verpackung einer Austrageinrichtung 40 zugeführt, mit der die Partikel vereinzelt und abgegeben werden. Hierzu weist die Austrageinrichtung einen nachfolgend näher beschriebenen Öffnungsmechanismus zum Öffnen der Verpackung der Partikel auf, wobei mit dem Öffnungsmechanismus die beiden miteinander verbundenen Filmstreifen 14 und 24 wieder voneinander gelöst werden. Nach dem Öffnen der Verpackung werden die an der Innenseite der Verpackung bzw. der beiden Filmstreifen 14 und 24 anhaftenden Partikel durch eine Düsenanordnung 42 zuerst abgesaugt und anschließend in vereinzelter Form aus einem Austragrohr 44 abgegeben. Sowohl das Absaugen der Partikel von der Innenseite der Filmstreifen 14 und 24 wie auch die Abgabe der Partikel in vereinzelter Form erfolgt mittels Druckluft, die über eine Zuführung 46 in die Düsenanordnung 42 der Austrageinrichtung 40 geführt wird.

Wie Fig. 2 verdeutlicht, werden die Partikel von der Innenseite der geöffneten Verpackung bzw. der wieder getrennten Filmstreifen 14 und 24 mit Hilfe des Bernoulli-Effekts abgesaugt, indem die durch die Zuführung 46 zugeführte Druckluft durch eine Blendenöffnung 48 geführt wird, so dass hinter der Blendenöffnung 48 ein Unterdruck entsteht, der die einzelnen Partikel von den Filmstreifen 14 und 24 absaugt und anschließend durch das Austragrohr 44 abgibt.

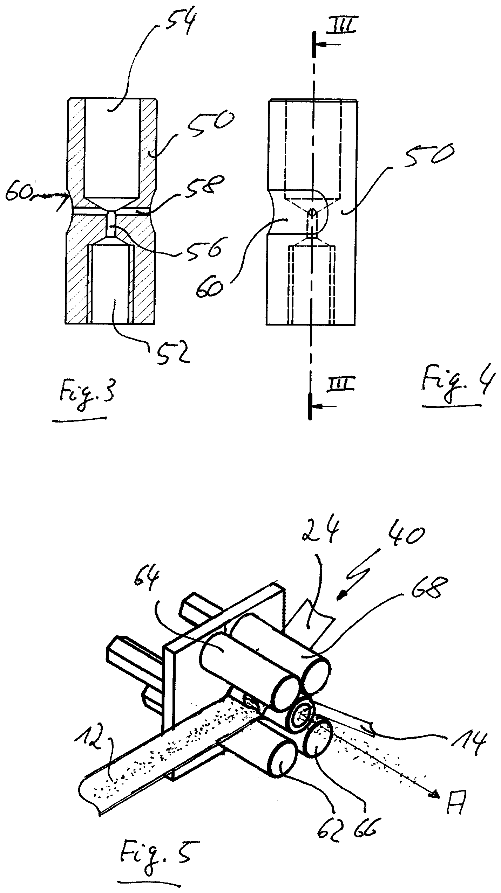

Wie die Fig. 3 und Fig. 4 verdeutlichen, sind bei dem dargestellten Ausführungsbeispiel die Düsenanordnung 42, die Blendenöffnung 48 und das Austragrohr 44 durch ein einziges rohrförmiges Bauteil 50 gebildet, das mit mehreren Bohrungen versehen ist. Das Bauteil 50 ist grundsätzlich zylinderförmig ausgebildet und weist an seiner Unterseite eine Bohrung, beim dargestellten Ausführungsbeispiel eine Gewindebohrung 52 auf, in die ein Anschluss für die Zuführung 46 für Druckluft einführbar bzw. einschraubbar ist. Von der gegenüberliegenden Seite des Bauteils 50 aus ist eine koaxiale Austragsbohrung 54 eingebracht, durch welche die vereinzelten Partikel aus der Austrageinrichtung abgegeben werden. Die Durchmesser der Gewindebohrung 52 und der Austragsbohrung 54 sind im Wesentlichen gleich, wobei beide Bohrungen 52 und 54 durch eine Blendenbohrung 56 mit wesentlich kleinerem Durchmesser miteinander verbunden sind. Quer zur Blendenbohrung 56 erstreckt sich durch das Bauteil 50 hindurch eine Ansaugbohrung 58, die etwa den gleichen Durchmesser wie die Blendenbohrung 56 aufweist und durch die Partikel in vereinzelter Form von den Filmstreifen 14 und 24 abgesaugt werden. In der Mitte des Bauteils 50 ist ferner an dessen äußerer Mantelfläche eine rinnenförmige Vertiefung 60 vorgesehen, die sich über etwas mehr als den halben Umfang des Bauteils 50 erstreckt, so dass die beiden Öffnungen der Ansaugbohrung 58 in die rinnenförmige Vertiefung 60 münden.

Im Betrieb wird über die Gewindebohrung 52 Druckluft in das Bauteil 50 gegeben, die durch die Blendenbohrung 56 und die Austragsbohrung 54 ausströmt. Hierbei entsteht im Bereich der Ansaugbohrung 58 und der Vertiefung 60 ein Unterdruck, mit dem die Partikel von der geöffneten Verpackung bzw. den wieder voneinander gelösten Filmstreifen 14 und 24 abgesaugt werden können, was in Fig. 5 näher dargestellt ist.

Fig. 5 zeigt in perspektivischer Ansicht die Austrageinrichtung 40, in welcher die beiden zunächst miteinander noch verbundenen Filmstreifen 14 und 24, zwischen denen sich die allergenen Partikel befinden, zugeführt werden. Die aus den beiden Filmstreifen bestehende Verpackung wird anschließend geöffnet, indem der untere Filmstreifen 14 und der obere Filmstreifen 24, die beide quer zur Längserstreckung des Bauteils 50 verlaufen, das Bauteil 50 umschlingen. Die Führung erfolgt durch beim dargestellten Ausführungsbeispiel insgesamt vier Führungsstifte 62, 64, 66 und 68, die dafür sorgen, dass die Filmstreifen 14 und 24, nachdem sie voneinander getrennt worden sind, das Bauteil 50 eng umschlingen und nach Durchlaufen der Austrageinrichtung 40 zu Aufwickelvorrichtungen geführt werden können. Die beiden Filmstreifen 14 und 24 werden dabei mit ihrer Innenseite über die rinnenförmige Vertiefung 60 des Bauteils 50 geführt, so dass alle zwischen den beiden Filmstreifen befindlichen Partikel vom Unterdruck erfasst werden und über die Ansaugbohrung 58 angesaugt und von dort über die Austragsbohrung 54 ausgetragen werden. Hierdurch kann eine Abgabe der Partikel in vereinzelter Form in Richtung des Pfeils A in Fig. 5 erreicht werden.

Mit der vorstehend beschriebenen Vorrichtung kann das eingangs geschilderte Verfahren zur kontrollierten Abgabe von allergenen Partikeln durchgeführt werden, bei dem die bevorrateten Partikel der Messstation 30 kontinuierlich zugeführt werden, wobei die Menge an zugeführten Partikeln in der Messstation 30 kontinuierlich erfasst wird. Das Erfassen kann insbesondere durch Zählen der zugeführten Partikel erfolgen, wobei auch Bilddaten der Partikel erfasst und abgespeichert werden können. Das Zuführen der Partikel zu der Messstation erfolgt bevorzugt in einer streifenförmigen Anordnung, was durch die beim dargestellten Ausführungsbeispiel aus zwei Filmstreifen bestehende Verpackung erleichtert ist. Die Partikel werden beim dargestellten Ausführungsbeispiel noch in der Verpackung befindlich durch die Messstation 30 hindurchgeführt und auch in der verschlossenen Verpackung in die Austrageinrichtung 40 eingebracht, wobei die Verpackung erst in der Austrageinrichtung 40 geöffnet wird. Die Partikel können dann in der Austrageinrichtung 40 von der Innenseite der geöffneten Verpackung abgesaugt werden, wobei hierbei bevorzugt zwei Filmstreifen eine Absaugeinrichtung derart umschlingen, dass die sich zwischen den Filmstreifen befindlichen Partikel weitgehend bzw. nahezu vollständig von den Filmstreifen abgesaugt werden. Die Abgabe der mittels Druckluft und Unterdruck abgesaugten Partikel erfolgt ebenfalls bevorzugt mittels Druckluft. Zur Gewährleistung einer konstanten Austragsmenge kann die Geschwindigkeit, mit der die Partikel zu der Austrageinrichtung überführt werden, in Abhängigkeit von der erfassten Menge an Partikeln geregelt werden. Hierzu kann es vorteilhaft sein, wenn die Partikel in der Messstation gezählt werden und die Zuführgeschwindigkeit in Abhängigkeit von der Zahl der erfassten Partikel geregelt wird.

Zur Verwendung in dem vorstehend beschriebenen Verfahren eignen sich grundsätzlich beliebige Partikel, insbesondere allergene Partikel, beispielsweise natürliche oder vorbehandelte Pollen. Hierbei können die Pollen bereits in vereinzelter Form insbesondere im Wesentlichen einlagig zwischen zwei Streifen eingebettet werden, so dass das Erfassen bzw. Zählen der einzelnen Partikel bzw. Pollen erleichtert ist.

## Patentansprüche

1. Verfahren zur kontrollierten Abgabe von allergenen Partikeln, umfassend die folgenden Schritte:
kontinuierliches Zuführen von bevorrateten Partikeln (12) zu einer Messstation (30);
kontinuierliches Erfassen der Menge an zugeführten Partikeln in der Messstation (30),
Überführen der erfassten Partikel von der Messstation (30) zu einer Austrageinrichtung (40), und
Abgabe der überführten Partikel aus der Austrageinrichtung (40), insbesondere in einen Raum.

2. Verfahren nach Anspruch 1,
**dadurch gekennzeichnet, dass**
die Partikel der Messstation (30) in einer streifenförmigen Anordnung zugeführt werden.

3. Verfahren nach Anspruch 1 und/oder 2,
**dadurch gekennzeichnet, dass**
die Partikel der Messstation (30) einlagig zugeführt werden.

4. Verfahren nach zumindest einem der vorstehenden Ansprüche,
**dadurch gekennzeichnet, dass**
die Partikel der Messstation (30) in einer Verpackung (14, 24) zugeführt und insbesondere in der Verpackung (14, 24) durch die Messstation (30) hindurchgeführt werden.

5. Verfahren nach zumindest einem der vorstehenden Ansprüche,
**dadurch gekennzeichnet, dass**
die Partikel in einer verschlossenen Verpackung (14, 24) in die Austrageinrichtung (40) eingebracht werden, und dass die Verpackung in der Austrageinrichtung geöffnet wird.

6. Verfahren nach zumindest einem der vorstehenden Ansprüche,
**dadurch gekennzeichnet, dass**
die Partikel in einer streifenförmigen Verpackung (14, 24) zugeführt werden,
die insbesondere zwei, vorzugsweise transparente oder transluzente, Filmstreifen umfasst.

7. Verfahren nach zumindest einem der vorstehenden Ansprüche,
**dadurch gekennzeichnet, dass**
die Partikel in der Austrageinrichtung (40) von der Innenseite einer geöffneten Verpackung abgesaugt werden.

8. Verfahren nach zumindest einem der vorstehenden Ansprüche,
**dadurch gekennzeichnet, dass**
die Partikel mittels Druckluft in der Austrageinrichtung (40) vereinzelt und aus der Austrageinrichtung abgegeben werden.

9. Verfahren nach zumindest einem der vorstehenden Ansprüche,
**dadurch gekennzeichnet, dass**
die Geschwindigkeit, mit der die Partikel zu der Austrageinrichtung (40) überführt werden, in Abhängigkeit von der erfassten Menge an Partikeln gesteuert wird.

10. Verfahren nach zumindest einem der vorstehenden Ansprüche,
**dadurch gekennzeichnet, dass**
die Partikel in der Messstation (30) gezählt werden.

11. Verfahren nach zumindest einem der vorstehenden Ansprüche,
**dadurch gekennzeichnet, dass**
von den Partikeln in der Messstation (30) Bilddaten erfasst werden.

12. Vorrichtung zur kontrollierten Abgabe von allergenen Partikeln, umfassend:
eine Zuführvorrichtung (36) zum kontinuierlichen Zuführen von bevorrateten Partikeln zu einer Messstation (30);
eine Messstation (30), in der die Menge an zugeführten Partikeln (12) kontinuierlich erfasst wird,
eine Austrageinrichtung (40) zur Abgabe der erfassten Partikel, insbesondere in einen Raum.

13. Vorrichtung nach Anspruch 12,
**dadurch gekennzeichnet, dass**
die Messstation (30) ein Mikroskop (32) aufweist, und/oder dass die Messstation (30) eine Zähleinrichtung (34) aufweist, mit der die Partikel gezählt werden.

14. Vorrichtung nach zumindest einem der vorstehenden Ansprüche 12 oder 13,
**dadurch gekennzeichnet, dass**
die Austrageinrichtung (40) einen Öffnungsmechanismus zum Öffnen einer Verpackung (14, 24) der Partikel (12) aufweist.

15. Vorrichtung nach zumindest einem der vorstehenden Ansprüche 12 - 14,
**dadurch gekennzeichnet, dass**
die Austrageinrichtung (40) eine Absaugeinrichtung aufweist, welche die Partikel von der Innenseite einer geöffneten Verpackung (14, 24) absaugt.

16. Vorrichtung nach zumindest einem der vorstehenden Ansprüche 12 - 15,
**dadurch gekennzeichnet, dass**
eine Steuereinrichtung (37) vorgesehen ist, welche die Geschwindigkeit der Zuführvorrichtung (36) in Abhängigkeit von der erfassten Menge an Partikeln steuert, und/oder dass die Austrageinrichtung (40) eine Düsenanordnung (42) aufweist, mit der mittels Druckluft Partikel angesaugt und in vereinzelter Form abgegeben werden.

17. Verpackung mit bevorrateten allergenen Partikeln zur Verwendung in einem Verfahren oder einer Vorrichtung nach zumindest einem der vorstehenden Ansprüche, umfassend zwei transparente oder transluzente und miteinander verbundene Filmstreifen (14, 24), zwischen denen sich in einer streifenförmigen und einlagigen Anordnung allergene Partikel (12) befinden.

18. Verpackung nach Anspruch 17,
**dadurch gekennzeichnet, dass**
die Partikel (12) in der streifenförmigen Anordnung zufällig verteilt sind.

19. Verfahren zur Herstellung einer Verpackung mit bevorrateten allergenen Partikeln, bei dem allergene Partikel (12) zwischen zwei transparenten oder transluzenten Film- oder Folienstreifen (14, 24) streifenförmig oder linienförmig und einlagig angeordnet werden, woraufhin die beiden Film- oder Folienstreifen (14, 24) zumindest an ihren Rändern miteinander verbunden werden, so dass die Partikel zwischen den Film- oder Folienstreifen (14, 24) eingeschlossen sind.

## Claims

1. A method for the controlled delivery of allergenic particles, comprising the following steps:
continuously supplying stored particles (12) to a measurement station (30);
continuously detecting the quantity of supplied particles in the measurement station (30);
transferring the detected particles from the measurement station (30) to a dispensing device (40); and
delivering the transferred particles from the dispensing device (40), in particular into a space.

2. A method in accordance with claim 1,
**characterized in that**
the particles are supplied to the measurement station (30) in a strip-shaped arrangement.

3. A method in accordance with claim 1 and/or claim 2,
**characterized in that**
the particles are supplied to the measurement station (30) in one layer.

4. A method in accordance with at least one of the preceding claims,
**characterized in that**
the particles are supplied to the measurement station (30) in a packaging (14, 24) and are in particular conducted through the measurement station (30) in the packaging (14, 24).

5. A method in accordance with at least one of the preceding claims,
**characterized in that**
the particles are introduced into the dispensing device (40) in a closed packaging (14, 24); and **in that** the packaging is opened in the dispensing device.

6. A method in accordance with at least one of the preceding claims,
**characterized in that**
the particles are supplied in a strip-shaped packaging (14, 24) which in particular comprises two film strips, preferably transparent or translucent film strips.

7. A method in accordance with at least one of the preceding claims,
**characterized in that**
the particles are sucked off from the inner side of an opened packaging in the dispensing device (40).

8. A method in accordance with at least one of the preceding claims,
**characterized in that**
the particles are isolated in the dispensing device (40) and are delivered from the dispensing device by means of compressed air.

9. A method in accordance with at least one of the preceding claims,
**characterized in that**
the speed at which the particles are transferred to the dispensing device (40) is controlled in dependence on the detected quantity of particles.

10. A method in accordance with at least one of the preceding claims,
**characterized in that**
the particles are counted in the measurement station (30).

11. A method in accordance with at least one of the preceding claims,
**characterized in that**
image data of the particles are acquired in the measurement station (30).

12. An apparatus for the controlled delivery of allergenic particles, comprising:
a feed apparatus (36) for the continuous supply of stored particles to a measurement station (30);
a measurement station (30) in which the quantity of supplied particles (12) is continuously detected;
a dispensing device (40) for delivering the detected particles, in particular into a space.

13. An apparatus in accordance with claim 12,
**characterized in that**
the measurement station (30) has a microscope (32); and/or **in that** the measurement station (30) has a counting device (34) with which the particles are counted.

14. An apparatus in accordance with at least one of the preceding claims 12 or 13,
**characterized in that**
the dispensing device (40) has an opening mechanism for opening a packaging (14, 24) of the particles (12).

15. An apparatus in accordance with at least one of the preceding claims 12 to 14,
**characterized in that**
the dispensing device (40) has a suction device which sucks the particles from the inner side of an opened packaging (14, 24).

16. An apparatus in accordance with at least one of the preceding claims 12 to 15,
**characterized in that**
a control device (37) is provided which controls the speed of the feed apparatus (36) in dependence on the detected quantity of particles; and/or in that the dispensing device (40) has a nozzle arrangement (42) with which particles are sucked up and are delivered in isolated form by means of compressed air.

17. A packaging with stored allergenic particles for use in a method and/or in an apparatus in accordance with at least one of the preceding claims, comprising two transparent or translucent film strips (14, 24) which are connected to one another and between which allergenic particles (12) are located in a strip-shaped and single-layer arrangement.

18. A packaging in accordance with claim 17,
**characterized in that**
the particles (12) are randomly distributed in the strip-shaped arrangement.

19. A method for manufacturing a packaging with stored allergenic particles, in which allergenic particles (12) are arranged in strip shape or in linear shape between two transparent or translucent film strips or foil strips (14, 24), whereupon the two film strips or foil strips (14, 24) are connected to one another at least at their margins so that the particles are enclosed between the film strips or foil strips (14, 24).

## Revendications

1. Procédé de distribution contrôlée de particules allergènes, comprenant les étapes suivantes :
- on amène en continu des particules approvisionnées (12) à un poste de mesure (30) ;
- on détecte en continu la quantité de particules amenées dans le poste de mesure (30),
- on transfère les particules détectées depuis le poste de mesure (30) vers un moyen d'extraction (40), et
- on distribue les particules transférées depuis le moyen d'extraction (40), en particulier dans un local.

2. Procédé selon la revendication 1,
**caractérisé en ce que**
on amène les particules en une disposition en forme de bande au poste de mesure (30).

3. Procédé selon la revendication 1 et/ou 2,
**caractérisé en ce que**
on amène les particules en une seule couche au poste de mesure (30).

4. Procédé selon l'une des revendications précédentes,
**caractérisé en ce que**
on amène les particules dans un emballage (14, 24) au poste de mesure (30) et on les fait passer à travers le poste de mesure (30) en particulier dans l'emballage (14, 24).

5. Procédé selon l'une des revendications précédentes,
**caractérisé en ce que**
on amène les particules dans un emballage fermé (14, 24) au moyen d'extraction (40) et **en ce que** l'on ouvre l'emballage dans le moyen d'extraction.

6. Procédé selon l'une des revendications précédentes,
**caractérisé en ce que**
on amène les particules dans un emballage (14, 24) en forme de bande qui comprend en particulier deux bandes en film de préférence transparentes ou translucides.

7. Procédé selon l'une des revendications précédentes,
**caractérisé en ce que**
on aspire les particules dans le moyen d'extraction (40) depuis le côté intérieur d'un emballage ouvert.

8. Procédé selon l'une des revendications précédentes,
**caractérisé en ce que**
on individualise les particules par de l'air comprimé dans le moyen d'extraction (40) et on les distribue depuis le moyen d'extraction.

9. Procédé selon l'une des revendications précédentes,
**caractérisé en ce que**
la vitesse à laquelle on transfère les particules vers le moyen d'extraction (40) est commandée en fonction de la quantité détectée de particules.

10. Procédé selon l'une des revendications précédentes,
**caractérisé en ce que**
on compte les particules dans le poste de mesure (30).

11. Procédé selon l'une des revendications précédentes,
**caractérisé en ce que**
on saisit des données d'image des particules dans le poste de mesure (30).

12. Dispositif de distribution contrôlée de particules allergènes, comprenant :
un dispositif d'amenée (36) pour amener en continu des particules approvisionnées vers un poste de mesure (30) ;
un poste de mesure (30) dans lequel la quantité de particules amenées (12) est détectée en continu,
un moyen d'extraction (40) pour distribuer les particules détectées, en particulier dans un local.

13. Dispositif selon la revendication 12,
**caractérisé en ce que**
le poste de mesure (30) comprend un microscope (32) et/ou **en ce que** le poste de mesure (30) comprend un moyen de comptage (34) à l'aide duquel les particules sont comptées.

14. Dispositif selon l'une des revendications précédentes 12 ou 13,
**caractérisé en ce que**
le moyen d'extraction (40) comprend un mécanisme d'ouverture pour ouvrir un emballage (14, 24) des particules (12).

15. Dispositif selon l'une des revendications précédentes 12 à 14,
**caractérisé en ce que**
le moyen d'extraction (40) comprend un moyen d'aspiration qui aspire les particules depuis le côté intérieur d'un emballage ouvert (14, 24).

16. Dispositif selon l'une des revendications précédentes 12 à 15,
**caractérisé en ce que**
il est prévu un moyen de commande (37) qui commande la vitesse du dispositif d'amenée (36) en fonction de la quantité détectée de particules, et/ou en ce que le moyen d'extraction (40) comprend un agencement de buse (42) à l'aide duquel des particules sont aspirées par de l'air comprimé et distribuées sous forme individualisée.

17. Emballage avec des particules allergènes approvisionnées pour l'utilisation dans un procédé ou dans un dispositif selon l'une au moins des revendications précédentes,
comprenant deux bandes en film (14, 24) transparentes ou translucides et reliées l'une à l'autre, entre lesquelles se trouvent des particules allergènes (12) en une disposition en forme de bande et en une seule couche.

18. Emballage selon la revendication 17,
**caractérisé en ce que**
les particules (12) sont réparties de façon aléatoire dans la disposition en forme de bande.

19. Procédé de réalisation d'un emballage avec des particules allergènes approvisionnées, dans lequel on agence des particules allergènes (12) en forme de bande ou en forme linéaire et en une seule couche entre deux bandes en film ou en feuille (14, 24) transparentes ou translucides, suite à quoi on relie les deux bandes en film ou en feuille (14, 24) l'une à l'autre au moins à leurs bords, de telle sorte que les particules sont enfermées entre les bandes en film ou en feuille (14, 24).
